# EUROPEAN PATENT SPECIFICATION

(11) **EP 2 663 859 B1**
(45) Date of publication and mention of the grant of the patent: **23.01.2019**
(21) Application number: 11855313.0
(22) Date of filing: 02.11.2011
(51) Int. Cl.: G01N 33/28, G01N 30/72

(54) **SYSTEM AND METHOD FOR PERFORMING GEOCHRONOLOGY**
SYSTEM UND VERFAHREN ZUR DURCHFÜHRUNG VON GEOCHRONOLOGIE
SYSTÈME ET PROCÉDÉ POUR EFFECTUER UNE GÉOCHRONOLOGIE

(30) Priority: 14.01.2011 US 201161432660 P
(43) Date of publication of application: 20.11.2013
(73) Proprietor: ExxonMobil Upstream Research Company, Spring TX 77389 (US)
(72) Inventor: DREYFUS, Sebastien, L., Houston TX 77008 (US); POTTORF, Robert, J., Houston TX 77055 (US)
(74) Representative: ExxonMobil Chemical Europe Inc.
(86) International application number: PCT/US2011/058982
(87) International publication number: WO 2012/096710

(56) References cited:
- US-A- 5 119 315
- US-A- 5 288 695
- US-A- 5 593 883
- US-A1- 2002 193 648
- US-A1- 2008 234 945
- US-A1- 2009 042 304
- US-B1- 7 151 447
- ALEXANDER V. IGNATIEV ET AL: "A continuous flow mass spectrometry technique of argon isotope measurement for K/Ar geochronology", RAPID COMMUNICATIONS IN MASS SPECTROMETRY., vol. 23, no. 16, 30 August 2009 (2009-08-30), pages 2403-2410, XP055320918, GB ISSN: 0951-4198, DOI: 10.1002/rcm.4017
- SELBY ET AL: "Re-Os elemental and isotopic systematics in crude oils", GEOCHIMICA ET COSMOCHIMICA ACTA, PERGAMON PRESS, NEW YORK, NY, US, vol. 71, no. 2, 27 December 2006 (2006-12-27), pages 378-386, XP005816431, ISSN: 0016-7037, DOI: 10.1016/J.GCA.2006.09.005
- FRANK VANHAECKE ET AL: "The determination of strontium isotope ratios by means of quadrupole-based ICP-mass spectrometry: a geochronological case study", JOURNAL OF ANALYTICAL ATOMIC SPECTROMETRY, vol. 14, no. 11, 1 January 1999 (1999-01-01), pages 1691-1696, XP055320967, ISSN: 0267-9477, DOI: 10.1039/A905184H
- JACKSON S E ET AL: "The application of laser ablation-inductively coupled plasma-mass spectrometry to in situ U-Pb zircon geochronology", CHEMICAL GEOLOGY, ELSEVIER SCIENCE PUBLISHER B.V., AMSTERDAM, NL, vol. 211, no. 1-2, 8 November 2004 (2004-11-08), pages 47-69, XP004572029, ISSN: 0009-2541, DOI: 10.1016/J.CHEMGEO.2004.06.017

## Description

### FIELD OF THE INVENTION

Embodiments of the present disclosure relate generally to the field of geochemistry. More particularly, the present disclosure relates to systems and methods for determining the age of a geologic material.

### BACKGROUND

This section is intended to introduce various aspects of the art, which may be associated with exemplary embodiments of the present disclosure. This discussion is believed to assist in providing a framework to facilitate a better understanding of particular aspects of the present invention. Accordingly, it should be understood that this section should be read in this light, and not necessarily as admissions of prior art.

Conventional techniques for dating of geologic materials, such as rocks, minerals, organic matter, oils or oil fractions, generally involve (i) isolating the material of interest; (ii) performing a series of wet chemistry steps in which the parent and daughter elements are separated, for example distillation and anion-exchange chromatography; and (iii) performing isotopic ratio measurements using mass spectrometry. The process is generally complex, time consuming, costly, and nonspecific with respect to where the parent/daughter elements are bound in the sample. For example, results published to date on rhenium-osmium (Re-Os) isotopic analysis of oils and oil fractions appear to report only the bulk isotopic composition of petroleum fluids, with no distinction between isotopic signatures for organic and inorganic species. Such convention techniques are described for example in SELBY ET AL: "Re-Os elemental and isotopic systematics in crude oils",GEOCHIMICA ET COSMOCHIMICA ACTA, PERGAMON PRESS, NEW YORK, NY, US, vol. 71, no. 2, 27 December 2006 (2006-12-27), pages 378-386 and ALEXANDER V. IGNATIEV ET AL: "A continuous flow mass spectrometry technique of argon isotope measurement for K/Ar geochronology",RAPID COMMUNICATIONS IN MASS SPECTROMETRY., vol. 23, no. 16, 30 August 2009 (2009-08-30), pages 2403-2410. Elements within organic species found in oils and oil fractions (e.g., Re, Os, U, Th, Pb, Rb, Sr) are related directly to the oil source rock and can therefore be used as radiometric chronometers for petroleum generation/expulsion dating. These metal-bearing organic species are easily contaminated by inorganic compounds within inorganic fluids and solids mixed with the oils, resulting in erroneous ages. Without deconvolution of the different species, the geochronologic results may be ambiguous or in error.

### SUMMARY

Accordingly, the present disclosure provides a system according to claim 11 and a method according to claim 1 for determining the geologic age of a sample without the use of conventional wet-chemistry preparation techniques. By eliminating the need for wet-chemistry techniques, the overall expense, inconvenience, delay, and/or potential inaccuracies of sample preparation may be reduced. Also, the present disclosure provides a computer readable medium according to claim 10.

Other features and advantages of the present disclosure will be readily apparent upon consideration of the following description in conjunction with the accompanying drawings.

### BRIEF DESCRIPTION OF THE DRAWINGS

Figure 1 is a simplified block diagram of a system in accordance with an embodiment of the present invention;
Figure 2 is a flow diagram of a method in accordance with an embodiment of the present invention;
Figure 3 is an exemplary plot of intensity-versus-time data sets in accordance with an embodiment of the present invention; and
Figure 4 is an exemplary isochronic plot in accordance with an embodiment of the present invention.

### DETAILED DESCRIPTION DEFINITIONS

Various terms as used herein are defined below. To the extent a term used in a claim is not defined below, it should be given the definition persons in the pertinent art have given that term in the context in which it is used.

As used herein, "a" or "an" entity refers to one or more of that entity. As such, the terms "a" (or "an"), "one or more", and "at least one" can be used interchangeably herein unless a limit is specifically stated.

As used herein, the terms "comprising," "comprises," "comprise," and "comprised" are open- ended transition terms used to transition from a subject recited before the term to one or more elements recited after the term, where the element or elements listed after the transition term are not necessarily the only elements that make up the subject.

As used herein, the terms "containing," "contains," and "contain" have the same open-ended meaning as "comprising," "comprises," and "comprise."

As used herein, the terms "having," "has," and "have" have the same open-ended meaning as "comprising," "comprises," and "comprise."

As used herein, the terms "including," "includes," and "include" have the same open-ended meaning as "comprising," "comprises," and "comprise."

As used herein the term "aqueous" means water-based.

As used herein the phrase "associated with an organic fraction" refers to an element (e.g., nickel or vanadium) that (i) is part of an organic molecule such as a porphyrin (e.g., a nickel porphyrin); or (ii) is otherwise present with, and perhaps bound to, an organic compound.

As used herein the term "isotope" refers to one of two or more atoms with the same atomic number but with different numbers of neutrons. Elements found in petroleum may be present in a variety of isotopes. As a well-known example, naturally-occurring uranium often includes at least two isotopes: U-238, which has 92 protons and 146 neutrons; and, in much lower concentrations, U-235, which again has 92 protons but only 143 neutrons.

As used herein the term "marker" means, with respect to a given sample, one or more elements, isotopes, or compounds, that usually are not naturally present in a sample of interest. Measurement of various marker concentrations in organic and inorganic fractions of a sample may help (i) to track, for example, the efficiency of sample separation and/or (ii) to determine more precisely the amounts of the fractions recovered after sample preparation. See also Standard.

As used herein the term "petroleum" includes, but is not limited to: gases, oils, tars, bitumens, asphaltenes, and/or the like. Natural petroleum also typically includes various elements such as, for example, nickel (Ni), vanadium (V), molybdenum (Mo), iron (Fe), cobalt (Co), rhenium (Re), gallium (Ga), osmium (Os), uranium (U), thorium (Th), and/or lead (Pb).

As used herein the term "aqueous fluids" includes, but is not limited to: water based fluids, solubilized (i.e., fluid-borne) rock or mineral (i.e., product of the digestion of a rock or mineral), and/or the like.

As used herein the phrase "organic fractions of petroleum" refers to groups of organic compounds that are associated with natural petroleum such as asphaltenes, NSO (nitrogen-sulfur-oxygen) fractions, and/or the like.

As used herein the term "radiogenic" refers generally to an isotope of an element that is created by radioactive decay; for example, osmium-187 is a radiogenic isotope created by radioactive decay of rhenium-187.

As used herein the term "signatures" refers to the relative abundances, concentrations and/or ratios of various elements and elemental isotopes.

As used herein the term "standard" refers to a solid or liquid having a known (and, generally, a certified) composition comprising one or more markers, typically used as an internal tracer or reference point. An organic standard might be, for example, a certified trace-element concentration of uranium, thorium, bismuth, or specific isotopes thereof, in an organic solvent such as xylene. Similarly an inorganic standard might be, for example, a similar trace-element concentration in an aqueous (i.e., water-based) solution. See also Marker.

### DETAILED DESCRIPTION

In the following section, specific embodiments of the present invention are described in connection with preferred embodiments. However, to the extent that the following description is specific to a particular embodiment or a particular use, this is intended to be for exemplary purposes only. Accordingly, the invention is not limited to the specific embodiments described below, but rather, it includes all alternatives, modifications, and equivalents falling within the scope of the appended claims.

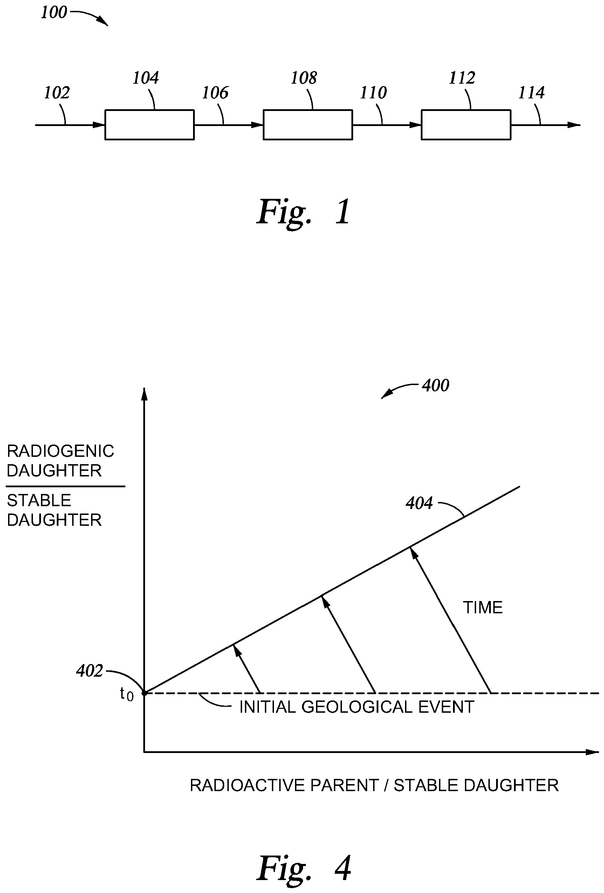

Referring to Figure 1, a diagram illustrating a system 100 that may be implemented in connection with the present invention is shown. The system generally includes a sample 102, a species generator 104 to receive the sample 102 and generate one or more species of interest 106, and/or a mass spectrometer 108 to receive the specie(s) of interest 106 and generate one or more data sets 110.

In general the sample 102 may be substantially petroleum or a mixture of petroleum and an aqueous fluid. However the sample 102 may be any substance or mixture capable of generating a species of interest 106 (i.e., an elemental compound associated with an organic fraction such as a vanadium porphyrin, nickel porphyrin, iron porphyrin, or the like). Similarly, the sample 102 may be generated using any appropriate mechanism.

The species generator 104 is a chromatographic column used to separate the sample 102 into the one or more species of interest 106. In such an embodiment the chromatographic column may be a liquid-chromatography column, such as a gel permeation chromatography column, or a gas chromatography column. However, any appropriate chromatographic column or set of columns may be implemented to meet the design criteria of a particular application. Furthermore, each chromatographic column may use any appropriate mechanism, such as polarity or molecular size, to separate the sample 102.

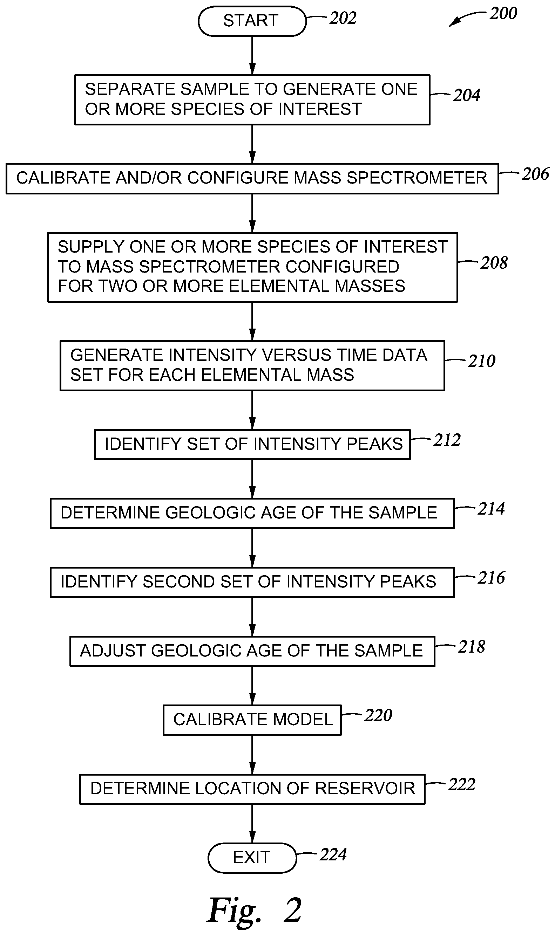

The mass spectrometer 108 may be any appropriate mass spectrometer or group of mass spectrometers, such as an inductively-coupled plasma mass spectrometer or more specifically a multi-collector inductively-coupled plasma mass spectrometer, that is capable of receiving the one or more species of interest 106 and generating a data set 110. The species of interest 106 may be supplied to (i.e., received by) the mass spectrometer 108 using any appropriate mechanism, such as laser ablation. Data sets 110 generally correspond to intensity over a duration of time. In at least one embodiment, data sets 110 may be used to determine (i.e., estimate) the geologic age of the sample 102 as discussed in connection with Figure 2. Optionally, in at least one embodiment, a processor or other computational device 112 may be implemented to generate user output data 114 corresponding to the geologic age of the sample 102.

Referring to Figure 2, a flow diagram of a method 200 for determining the geologic age of a sample of a naturally-occurring substance is shown. The method 200 may be advantageously implemented in connection with the system 100, described previously in connection with Figure 1, and/or any appropriate system to meet the design criteria of a particular application. The method 200 generally includes a plurality of blocks or steps (e.g., 202, 204, 206, and the like) that may be performed serially. As will be appreciated by one of ordinary skill in the art, the order of the steps shown in Figure 2 is exemplary and the order of one or more steps may be modified within the spirit and scope of the present invention. Additionally, the steps of the method 200 may be performed in at least one non-serial (or non-sequential) order, and one or more steps may be omitted to meet the design criteria of a particular application.

Block 202 represents an entry point into the method 200.

At block 204 a sample (e.g., 102) is separated into one or more species of interest (e.g., 106). In general the sample may be substantially petroleum or a mixture of petroleum and an aqueous fluid. However the sample may be any substance or mixture capable of generating a species of interest (i.e., an elemental compound associated with an organic fraction such as a vanadium porphyrin, nickel porphyrin, iron porphyrin, or the like).

The chromatographic column may be a liquid-chromatography column, such as a gel permeation chromatography column, or a gas chromatography column. However, any appropriate chromatographic column or set of columns may be implemented to meet the design criteria of a particular application. Furthermore, each chromatographic column may use any appropriate mechanism, such as polarity or molecular size, to separate the sample.

At block 206 a mass spectrometer (e.g., 108) may be calibrated for use in the method 200. In at least one embodiment the mass spectrometer may be optionally calibrated by adding a standard, such as an isotopic standard, to the sample or a portion of the sample. However, any appropriate calibration technique may be implemented to meet the design criteria of a particular embodiment.

Configuration of the mass spectrometer is generally performed at block 206 and generally includes configuring the mass spectrometer for two or more elemental masses, wherein each of the two or more elemental masses is included in at least one of the species of interest. For example, in at least one embodiment, the two or more elemental masses may be selected from the group consisting of radioactive-parent mass, radiogenic-daughter mass, and stable-daughter mass. In general, the term "elemental mass" refers to an isotope of an element of interest that is present in the sample.

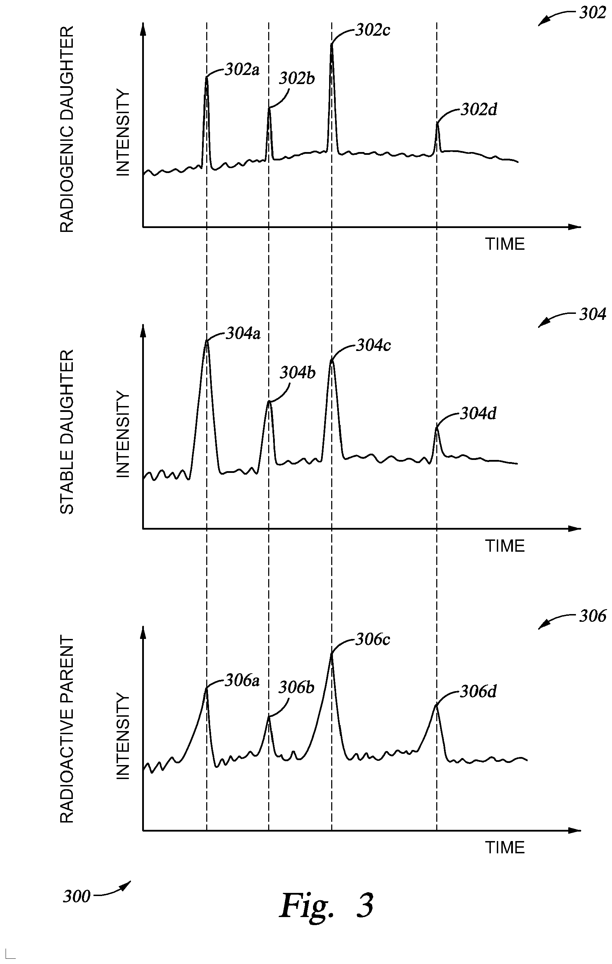

At blocks 208 and 210 the one or more species of interest may be supplied (block 208) to the mass spectrometer to generate (block 210) an intensity-versus-time data set (e.g., 110) for each of the two or more elemental masses. A non-limiting example having intensity-versus-time data sets corresponding to three elemental masses is provided in Figure 3. In general, each of the intensity-versus-time data sets includes one or more intensity peaks and each of the intensity peaks corresponds to a species of interest. Additionally, the species of interest may be supplied to the mass spectrometer 108 using any appropriate mechanism, such as laser ablation.

At block 212 a set of intensity peaks is identified. The set includes members from each of at least two of the intensity-versus-time data sets. In general, each member corresponds to the same species of interest. In at least one embodiment of the present invention, intensity peaks corresponding to the same species of interest (e.g., 302a, 304a and 306a of Figure 3) may have substantially identical elution times (i.e., the time that the elemental mass in question, as part of a given species of interest, is outputted during the separation process).

At block 214 the geologic age of the sample may be determined using ratios of the members of the set of intensity peaks. For example, Figure 4 illustrates a geologic age determination in an embodiment where an intensity-versus-time data set is generated for three elemental masses (i.e., radiogenic daughter, stable daughter and radioactive parent). The ratio of a radiogenic daughter to a stable daughter may be plotted against the ratio of a radioactive parent to the stable daughter and the resulting curve/line 404 may be used to determine the geologic age of the sample. While plots corresponding to three elemental masses were used to construct the curve 404 of Figure 4, it may be appreciated that a similar curve could be generated using only two elemental masses (e.g., radioactive parent and radiogenic daughter) as the elemental mass corresponding to stable daughter is a common divisor.

Block 216 represents the optional step of identifying a second set of intensity peaks having members from each of at least two of the intensity-versus-time data sets. In general the members of the second set (e.g., the set 302b, 304b, 306b; or the set 302b and 306b in the case where plot 304 was not generated) correspond to another one of the species of interest (i.e., a second species of interest).

At block 218 the determined geologic age of the sample may be optionally adjusted based on ratios of the members of the second set in a similar manner as the geologic age of the sample was determined at block 214.

At block 220 the determined (from block 214) and/or adjusted (from block 218) geologic age may be optionally used to calibrate a model such as a basin model.

Similarly, block 222 represents the optional step of using the determined (from block 214) and/or adjusted (from block 218) geologic age to determine a location of a petroleum reserve. In at least one embodiment, determining the location of the petroleum reserve may further include the use of an earth model running on a computer.

Block 224 represents an exit point out of the method 200.

Turning now to Figure 3 a non-limiting example of intensity-versus-time data sets 300 that may be generated by a mass spectrometer (e.g., 108) in accordance with the method 200 of Figure 2 is shown. In this particular example, the intensity of a radiogenic daughter is plotted against time at 302, the intensity of a stable daughter is plotted against time at 304, and the intensity of the radioactive parent is plotted against time at 306. It may be noted that the count of a given elemental mass as detected and recorded by, for example, a mass spectrometer is generally proportional to the concentration of the elemental mass and is typically referred to as the "intensity" of the elemental mass. Furthermore, it should be understood that while Figure 3 shows the intensity-versus-time data set for each of three elemental masses, other embodiments may include intensity-versus-time data sets for only two elemental masses. As discussed previously in connection with block 214 of Figure 2, proper selection of elemental masses may provide for determination of the geologic age of a sample with as few as two intensity-versus-time data sets. Once again, corresponding peaks (e.g., 302a, 304a and 306a) generally relate to the same species of interest.

Figure 4 shows a non-limiting example of a plot 400 to determine the geologic age of a sample (e.g., 102) using ratios of a set of intensity peaks selected from data sets (e.g., 300). In this particular example, the ratio of a radiogenic daughter to a stable daughter is plotted against the ratio of a radioactive parent to the stable daughter to generate the isochron 404. Once again, since stable daughter is a common divisor an isochron having the same slope as curve 404 may be generated by plotting radiogenic daughter against radioactive parent (i.e., omitting stable daughter data).

Point 402 corresponds to time value zero (i.e., the time of the initial geologic event). The slope of the resulting curve 404 may be generally defined by the equation: *slope* = *e*^{*λt*-1} where λ equals a decay constant and t is time in millions of years. As will be generally appreciated by one skilled in the art having the benefit of this disclosure, the slope of the curve 404 corresponds to the age of the related sample.

In at least one embodiment, the radioactive parent and radiogenic daughter may be rhenium-187 and osmium-187, respectively. In such an embodiment the corresponding stable daughter mass would be osmium 188. Other radioactive parent/radiogenic daughter combinations may be U-235/Pb-207, U-238/Pb-206, and Th-232/Pb-208, for each of which Pb-204 is the common stable daughter mass; also Rb-87/Sr-87, for which Sr-86 is the stable daughter mass. However, any appropriate radioactive parent/ radiogenic daughter combination may be used to satisfy the design criteria of a particular application.

In accordance with various embodiments of the present invention, aspects of the methods described herein may be implemented as software programs running on a computer processor (e.g., 112). Dedicated hardware implementations including, but not limited to, application specific integrated circuits, programmable logic arrays and other hardware devices can likewise be constructed to implement one or more of the method steps described herein. Furthermore, alternative software implementations including, but not limited to, distributed processing or component/object distributed processing, parallel processing, or virtual machine processing can also be constructed to implement one or more of the method steps described herein.

## Claims

1. A method for determining the geologic age of a sample (102) of a naturally-occurring substance comprising:
using a chromatographic column (104) to separate the sample (102) into one or more species of interest (106) each having an elution time, the species of interest (106) being an elemental compound associated with an organic fraction;
supplying the one or more species of interest (106) to a mass spectrometer (108), the mass spectrometer configured for two or more elemental masses including radioactive-parent mass and radiogenic-daughter mass, wherein each of the selected two or more elemental masses are included in at least one of the one or more species of interest (106);
generating, via the mass spectrometer (108) supplied with the one or more species of interest (106), an intensity-versus-time data set (110) for each of the two or more elemental masses, wherein each of the intensity-versus-time data sets (110) includes one or more intensity peaks, each of the intensity peaks corresponding to one of the species of interest (106);
identifying a set of intensity peaks having members from each of at least two of the intensity-versus-time data sets (110), each member of the set of intensity peaks having substantially the same elution time, the members corresponding to a single species of interest (106); and
determining the geologic age of the sample (102) using ratios of the members of the set of intensity peaks.

2. The method of claim 1, wherein the mass spectrometer (108) is an inductively-coupled plasma mass spectrometer, preferably a multi-collector inductively-coupled plasma mass spectrometer.

3. The method of claim 1, wherein the sample (102) comprises at least one of petroleum and an aqueous fluid.

4. The method of claim 1, wherein said chromatographic column (104) being a liquid-chromatography column such as a gel permeation chromatography column or a gas chromatography column.

5. The method of claim 4, wherein the chromatographic column (104) separates the sample (102) using at least one of polarity and molecular size.

6. The method of claim 1, further including the step of calibrating the mass spectrometer (108) by adding an isotopic standard to the sample.

7. The method of claim 1, further including identifying a second set of intensity peaks having members from each of at least two of the intensity-versus-time data sets (110), the members of the second set corresponding to a second species of interest; and
adjusting the determined geologic age of the sample using ratios of the members of the second set to generate an adjusted geologic age of the sample.

8. The method of claim 7, further including using the adjusted geologic age to calibrate a basin model.

9. The method of claim 1, further including using the determined geologic age to calibrate a basin model.

10. A computer readable medium comprising non-transitory instructions which, when executed by a computer, cause a system according to claim 11 to execute the steps of the method of claim 1 or claim 7.

11. A system for determining the geologic age of a sample of a substance (102) comprising:
a species generator (104) configured to receive the sample and to generate one or more species of interest, the species generator (104) comprising a chromatographic column to separate the sample into one or more species of interest (106) each having an elution time, the species of interest (106) being an elemental compound associated with an organic fraction;
a mass spectrometer (108) configured to receive the one or more species of interest (106) and to generate an intensity-versus-time data set (110) for each of two or more predetermined elemental masses including radioactive-parent mass and radiogenic-daughter mass, wherein each of the intensity-versus-time data sets (110) includes one or more intensity peaks, each of the intensity peaks corresponding to one of the species of interest (106);
a processor configured to perform the following steps:
identifying a set of intensity peaks having members from each of at least two of
the intensity-versus-time data sets (110), the members corresponding to a single species of the one or more species of interest (106), each member of the set of intensity peaks having substantially the same elution time;
determining the geologic age of the sample (102) using ratios of the members of the set of intensity peaks; and optionally
identifying a second set of intensity peaks having members from each of at least two of the intensity-versus-time data sets, the members of the second set corresponding to a second species of interest selected from the one or more species of interest (106); and
adjusting the determined geologic age of the sample (102) using ratios of the members of the second set to generate an adjusted geologic age of the sample (102).

## Patentansprüche

1. Verfahren zum Bestimmen des geologischen Alters einer Probe (102) einer natürlich vorkommenden Substanz, bei dem
eine chromatographische Säule (104) verwendet wird, sodass die Probe (102) in ein oder mehrere zu bestimmende Teilchen (106), die jeweils eine Eluationszeit aufweisen, aufgetrennt wird, welches zu bestimmende Teilchen (106) eine Elementarverbindung ist, die von einer organischen Fraktion abgeleitet ist,
die ein oder mehreren zu bestimmenden Teilchen (106) in ein Massenspektrometer (108) eingebracht werden, welches Massenspektrometer für zwei oder mehr Elementarmassen, die radioaktive Ausgangsmasse und radiogene Tochtermasse enthalten, konfiguriert ist, wobei jede der ausgewählten zwei oder mehr Elementarmassen in mindestens einem der zu bestimmenden Teilchen (106) enthalten ist,
ein Intensität vs. Zeit Datensatz (110) für jede der zwei oder mehr Elementarmassen mittels des Massenspektrometers (108) erzeugt wird, in das eine oder mehrere zu bestimmende Teilchen (106) eingebracht werden, wobei jeder der Intensität vs. Zeit Datensätze (110) ein oder mehrere Intensitätspeaks enthält, wobei jeder der Intensitätspeaks einem der zu bestimmenden Teilchen (106) entspricht,
ein Intensitätspeak-Set, das Mitglieder von jedem der mindestens zwei Intensität vs. Zeit Datensätzen (110) aufweist, identifiziert wird, wobei jedes Mitglied des Intensitätspeak-Sets eine im Wesentlichen gleiche Eluationszeit aufweist, wobei die Mitglieder einem einzelnen zu bestimmenden Teilchen entsprechen (106), und
das geologische Alter der Probe (102) mittels Verhältnissen der Mitglieder der Intensitätspeak-Sets bestimmt wird.

2. Verfahren nach Anspruch 1, bei dem das Massenspektrometer (108) ein induktivgekoppeltes Plasmamassenspektrometer ist, vorzugsweise ein Multikollektor-induktivgekoppeltes Plasmamassenspektrometer.

3. Verfahren nach Anspruch 1, bei dem die Probe (102) mindestens eines von Benzin und wässrige Flüssigkeit umfasst.

4. Verfahren nach Anspruch 1, bei dem die chromatographische Säule (104) eine Flüssigkeitschromatographiesäule, wie eine Gelpermeationschromatograhiesäule, oder eine Gaschromatographiesäule ist.

5. Verfahren nach Anspruch 4, bei dem die chromatographische Säule (104) die Probe (102) unter Verwendung der Polarität und Molekulargröße auftrennt.

6. Verfahren nach Anspruch 1, bei dem ferner das Massenspektrometer (108) mittels Addition eines Isotopenstandards zu der Probe kalibriert wird.

7. Verfahren nach Anspruch 1, bei dem ferner ein zweites Intensitätspeak-Set identifiziert wird, das Mitglieder von jedem der mindestens zwei Intensität- vs. Zeit Datensets (110) aufweist, wobei die Mitglieder des zweiten Sets einem zweiten zu bestimmenden Teilchen entsprechen, und
das bestimmte geologische Alter der Probe mittels Verhältnissen der Mitglieder des zweiten Sets angepasst wird, sodass ein angepasstes geologisches Alter der Probe erzeugt wird.

8. Verfahren nach Anspruch 7, bei dem ferner ein Beckenmodell unter Verwendung des angepassten geologischen Alters kalibriert wird.

9. Verfahren nach Anspruch 1, bei dem ferner ein Beckenmodell unter Verwendung des bestimmten geologischen Alters kalibriert wird.

10. Computerlesbares Medium, das nicht-transistorische Instruktionen umfasst, das bewirkt, wenn es durch einen Computer ausgeführt wird, dass ein System gemäß Anspruch 11 die Stufen des Verfahrens gemäß Anspruch 1 oder Anspruch 7 ausführt.

11. System zur Bestimmung des geologischen Alters einer Probe einer Substanz (102), das
Teilchengenerator (104), der so konfiguriert ist, dass eine Probe erhalten wird und ein oder mehrere zu bestimmende Teilchen erzeugt werden, welcher Teilchengenerator (104) eine chromatographische Säule umfasst, sodass die Probe in ein oder mehrere zu bestimmende Teilchen (106) aufgetrennt wird, die jeweils eine Eluationszeit aufweisen, welches zu bestimmende Teilchen (106) eine Elementarverbindung ist, die von einer organischen Fraktion abgeleitet ist,
Massenspektrometer (108), das so konfiguriert ist, dass ein oder mehrere zu bestimmende Teilchen (106) erhalten werden und ein Intensität- vs. Zeit Datenset (110) für jede der zwei oder mehr vorbestimmten Elementarmassen, die radioaktive Ausgangsmasse und radiogene Tochtermasse enthalten, erzeugt wird, wobei jedes der Intensität- vs. Zeit Datensets (110) ein oder mehrere Intensitätspeaks enthält, jeder der Intensitätspeaks entspricht einem der zu bestimmenden Teilchen (106),
Prozessor, der so konfiguriert ist, dass
ein Set an Intensitätspeaks identifiziert wird, das Mitglieder von jedem der mindestens zwei der Intensität- vs. Zeit Datensets (110) aufweist, welche Mitglieder einem einzelnen Teilchen des einen oder der mehreren zu bestimmenden Teilchen entsprechen (106), wobei jedes Mitglied des Intensitätspeaks-Sets im Wesentlichen die gleiche Eluationszeit aufweist,
das geologische Alter der Probe (102) mithilfe Verhältnissen der Mitglieder der Intensitätspeaks-Sets bestimmt wird, und gegebenenfalls
ein zweites Intensitätspeaks-Set identifiziert wird, das Mitglieder von jedem der mindestens zwei der Intensität- vs. Zeit Datensets (110) aufweist, welche Mitglieder des zweiten Sets einem zweiten zu bestimmende Teilchen entsprechen, das von dem einem oder den mehreren zu bestimmende Teilchen (106) ausgewählt ist, und
das bestimmte geologische Alter der Probe (102) mittels Verhältnissen der Mitglieder des zweiten Sets angepasst wird, sodass ein angepasstes geologisches Alter der Probe (102) erzeugt wird,
umfasst.

## Revendications

1. Procédé de détermination de l'âge géologique d'un échantillon (102) d'une substance naturelle comprenant :
l'utilisation d'une colonne de chromatographie (104) pour séparer l'échantillon (102) en une ou plusieurs espèces d'intérêt (106) ayant chacune un temps d'élution, l'espèce d'intérêt (106) étant un composé élémentaire associé à une fraction organique ;
l'introduction de la ou des espèces d'intérêt (106) dans un spectromètre de masse (108), le spectromètre de masse étant configuré pour au moins deux masses élémentaires incluant une masse de parent radioactif et une masse de fils radiogénique, chacune des au moins deux masses élémentaires sélectionnées étant contenue dans la ou au moins une des espèces d'intérêt (106) ;
la génération, par le biais du spectromètre de masse (108) dans lequel la ou les espèces d'intérêt (106) ont été introduites, d'un ensemble de données d'intensité en fonction du temps (110) pour chacune des au moins deux masses élémentaires, chacun des ensembles de données d'intensité en fonction du temps (110) comportant un ou plusieurs pics d'intensité, chacun des pics d'intensité correspondant à une des espèces d'intérêt (106) ;
l'identification d'un ensemble de pics d'intensité ayant des éléments issus de chacun d'au moins deux des ensembles de données d'intensité en fonction du temps (110), chaque élément de l'ensemble de pics d'intensité ayant sensiblement le même temps d'élution, les éléments correspondant à une seule espèce d'intérêt (106) ; et
la détermination de l'âge géologique de l'échantillon (102) au moyen de proportions des éléments de l'ensemble de pics d'intensité.

2. Procédé de la revendication 1, dans lequel le spectromètre de masse (108) est un spectromètre de masse à plasma à couplage inductif, de préférence un spectromètre de masse à plasma à couplage inductif à multiples collecteurs.

3. Procédé de la revendication 1, dans lequel l'échantillon (102) comprend un fluide pétrolier et/ou aqueux.

4. Procédé de la revendication 1, dans lequel ladite colonne de chromatographie (104) est une colonne de chromatographie liquide telle qu'une colonne de chromatographie par perméation de gel ou une colonne de chromatographie gazeuse.

5. Procédé de la revendication 4, dans lequel la colonne de chromatographie (104) sépare l'échantillon (102) en utilisant la polarité et/ou la taille moléculaire.

6. Procédé de la revendication 1, comportant en outre l'étape d'étalonnage du spectromètre de masse (108) par ajout d'un étalon isotopique à l'échantillon.

7. Procédé de la revendication 1, comportant en outre l'identification d'un deuxième ensemble de pics d'intensité ayant des éléments issus de chacun d'au moins deux des ensembles de données d'intensité en fonction du temps (110), les éléments du deuxième ensemble correspondant à une deuxième espèce d'intérêt ; et
l'ajustement de l'âge géologique déterminé de l'échantillon au moyen de proportions des éléments du deuxième ensemble pour générer un âge géologique ajusté de l'échantillon.

8. Procédé de la revendication 7, comportant en outre l'utilisation de l'âge géologique ajusté pour étalonner un modèle de bassin.

9. Procédé de la revendication 1, comportant en outre l'utilisation de l'âge géologique déterminé pour étalonner un modèle de bassin.

10. Support lisible par ordinateur comprenant des instructions non transitoires qui, exécutées par un ordinateur, conduisent un système selon la revendication 11 à exécuter les étapes du procédé de la revendication 1 ou la revendication 7.

11. Système destiné à déterminer l'âge géologique d'un échantillon d'une substance (102) comprenant :
un générateur d'espèces (104) configuré pour recevoir l'échantillon et pour générer une ou plusieurs espèces d'intérêt, le générateur d'espèces (104) comprenant une colonne de chromatographie pour séparer l'échantillon en une ou plusieurs espèces d'intérêt (106) ayant chacune un temps d'élution, l'espèce d'intérêt (106) étant un composé élémentaire associé à une fraction organique ;
un spectromètre de masse (108) configuré pour recevoir la ou les espèces d'intérêt (106) et pour générer un ensemble de données d'intensité en fonction du temps (110) pour chacune d'au moins deux masses élémentaires prédéterminées incluant une masse de parent radioactif et une masse de fils radiogénique, chacun des ensembles de données d'intensité en fonction du temps (110) comportant un ou plusieurs pics d'intensité, chacun des pics d'intensité correspondant à une des espèces d'intérêt (106) ;
un processeur configuré pour effectuer les étapes suivantes :
identifier un ensemble de pics d'intensité ayant des éléments issus de chacun d'au moins deux des ensembles de données d'intensité en fonction du temps (110), les éléments correspondant à une seule espèce parmi la ou les espèces d'intérêt (106), chaque élément de l'ensemble de pics d'intensité ayant sensiblement le même temps d'élution ;
déterminer l'âge géologique de l'échantillon (102) en utilisant des proportions des éléments de l'ensemble de pics d'intensité ; et éventuellement
identifier un deuxième ensemble de pics d'intensité ayant des éléments issus de chacun d'au moins deux des ensembles de données d'intensité en fonction du temps, les éléments du deuxième ensemble correspondant à une deuxième espèce d'intérêt choisie parmi la ou les espèces d'intérêt (106) ; et
ajuster l'âge géologique déterminé de l'échantillon (102) en utilisant des proportions des éléments du deuxième ensemble pour générer un âge géologique ajusté de l'échantillon (102).
